Europäisches Patentamt

European Patent Office

Office européen des brevets

(19)

(11) **EP 0 831 758 B1**

(12) **EUROPÄISCHE PATENTSCHRIFT**

(45) Veröffentlichungstag und Bekanntmachung des Hinweises auf die Patenterteilung:
**29.08.2001  Patentblatt 2001/35**

(51) Int Cl.⁷: **A61F 2/30**
// A61F2/38

(21) Anmeldenummer: **96921985.6**

(22) Anmeldetag: **14.06.1996**

(86) Internationale Anmeldenummer:
**PCT/EP96/02566**

(87) Internationale Veröffentlichungsnummer:
**WO 97/00053 (03.01.1997 Gazette 1997/02)**

(54) **KÜNSTLICHES GELENK, INSBESONDERE ENDOPROTHESE ZUM ERSATZ NATÜRLICHER GELENKE**

ARTIFICIAL JOINT, IN PARTICULAR ENDOPROSTHESIS FOR REPLACING NATURAL JOINTS

ARTICULATION ARTIFICIELLE, NOTAMMENT ENDOPROTHESE DE REMPLACEMENT D'ARTICULATIONS NATURELLES

(84) Benannte Vertragsstaaten:
**CH DE FR GB IT LI**

(30) Priorität: **14.06.1995  DE 19521597**

(43) Veröffentlichungstag der Anmeldung:
**01.04.1998  Patentblatt 1998/14**

(73) Patentinhaber: **HJS Gelenk System GmbH**
**81925 München (DE)**

(72) Erfinder:
• **KUBEIN-MEESENBURG, Dietmar**
**D-37547 Kreiensen (DE)**
• **NÄGERL, Hans**
**D-37130 Gleichen (DE)**

(74) Vertreter: **Braun, Dieter, Dipl.-Ing. et al**
**Hagemann, Braun & Held**
**Patentanwälte,**
**Hildesheimer Strasse 133**
**30173 Hannover (DE)**

(56) Entgegenhaltungen:
EP-A- 0 498 586    DE-C- 4 202 717
US-A- 3 748 662    US-A- 3 798 679
US-A- 4 242 759

**Beschreibung**

Die vorliegende Erfindung betrifft ein künstliches Gelenk, insbesondere eine Endoprothese zum Ersatz natürlicher Gelenke, bestehend aus mindestens zwei künstlichen Gelenkteilen mit jeweils gekrümmten Artikulationsflächen, auf denen die Gelenkteile relativ zueinander artikulieren.

Ein derartiges künstliches Gelenk ist aus der US-A-3 798 679 bekannt. Bei diesem Gelenk findet zwischen den Gelenkteilen theoretisch ein Linien kontakt, in der Praxis und unter Druck jedoch ein Flächen kontakt statt.

Ein derartiges künstliches Gelenk ist ferner aus der deutschen Patentanmeldung P 42 02 717.9 bekannt. Hierbei besitzen die Gelenkflächen in zueinander senkrechten Ebenen, und zwar einer Längsebene und einer Querebene, unterschiedliche kreisförmige Schnittkonturen, wobei die Krümmungsverhältnisse der Artikulationsflächen in jeder der Ebenen konvex-konvex, konvex-konkav oder konkav-konkav sind, und die Gelenkgeometrie der Artikulationsflächen zueinander in jeder der beiden Ebenen durch eine Gelenkkette mit zwei Gelenkachsen (dimere Gelenkkette) bestimmt ist, die durch die Rotationszentren der Artikulationsflächen der jeweils zugehörigen Schnittkonturen verlaufen. Da die Artikulationsflächen dieses künstlichen Gelenks konvex-konkav, konkav-konkav bzw. konvex-konvex ausgebildet sind, entstehen im wesentlichen punktförmige Kraftübertragungsbereiche, wodurch erhöhte Flächenpressungen auf den Artikulationsflächen entstehen, die zu einem Materialabrieb führen können. Hierdurch kann die Lebensdauer dieser künstlichen Gelenke verkürzt werden. Um eine Verbesserung der Kraftübertragung zwischen den Artikulationsflächen der Gelenkteile zu erreichen, ist bei dem bekannten Gelenk vorgeschlagen, zwischen diesen jeweils einen Druckverteilungskörper anzuordnen, mit dem eine bessere und gleichmäßigere Kraftverteilung erreicht wird. Durch diesen Druckverteilungskörper erhöht sich jedoch die Anzahl der erforderlichen Gelenkteile des künstlichen Gelenks.

Der vorliegenden Erfindung liegt die Aufgabe zugrunde, ein künstliches Gelenk zu schaffen, bei dem punktuelle Kraftübertragungsbereiche vermieden werden und bei dem der Einbau von Druckverteilungskörpern nicht erforderlich ist, und das gleichzeitig eine optimale Anpassung an die Gegebenheiten des menschlichen Körpers im Einsatz als Endoprothese für ein natürliches menschliches Gelenk ermöglicht.

Erfindungsgemäß wird dies mit einem künstlichen Gelenk erreicht, das aus mindestens zwei künstlichen Gelenkteilen mit gekrümmten Artikulationsflächen besteht, wobei auf jeder der Artikulationsflächen eine kreisbogenförmige Kontaktlinie ausgebildet ist, die jeweils ein Teilabschnitt eines in einer Ebene liegenden Kontaktkreises mit einem Mittelpunkt M ist, wobei die Artikulationsflächen derart als Paar zueinander angeordnet sind, daß die Kontaktlinien aufeinander abrollen können sowie senkrecht zu der Ebene der Kontaktkreise durch deren Mittelpunkt verlaufende Achsen sich in einem Schnittpunkt S schneiden, sowie einseitig an die Kontaktlinien aus einer Vielzahl von geraden Berührungslinien gebildete, die Artikulationsflächen darstellende Regelflächen (ruled surfaces) angeformt sind, wobei die Berührungslinien auf momentanen Verbindungslinien der während der Abrollbewegung auftretenden momentanen Kontaktpunkte mit den momentanen Schnittpunkten liegen, welche sich durch eine Schwenkbewegung der Kontaktlinien mit einer Winkelgeschwindigkeit Q um eine gemeinsame Tangente der Kontaktlinien durch die momentanen Kontaktpunkte ergeben. Erfindungsgemäß wird demnach ein räumliches Getriebesystem gebildet. Hierbei wird der einzelnen Gelenkoberfläche jedes Gelenkteils (Gliedmaße) eine Kontaktlinie zugeordnet, wobei sich diese Kontaktlinien immer berühren und eine gemeinsame Kontakttangente besitzen. Während der Artikulation der Gelenkteile kann sich hierbei die Neigung der auf den Kontaktkreisen liegenden Kontaktlinien zueinander verändern, wobei sie eine Rotation um die im Kontaktpunkt jeweils verlaufende gemeinsame Kontakttangente durchführen, wobei dann die Kontaktkreis-Achsen eine variierende sphärische Anordnung zueinander zeigen. Da erfindungsgemäß im Bereich der Regelflächen gerade Berührungslinien zwischen den artikulierenden Regelflächen vorhanden sind, wobei diese Berührungslinien vom Kontaktpunkt der Kontaktlinien ausgehen, entsteht neben den Kontaktlinien ein Bereich der direkten Kraftübertragung, wobei linienförmige Kraftübertragungsbereiche durch die Berührungslinien vorhanden sind. Somit wird eine punktuelle Kraftübertragung vermieden, wodurch die Materialbelastung im Bereich der Regelflächen wesentlich gegenüber bekannten Gelenken verringert wird. Hierdurch ist es möglich, auch Materialien verwenden zu können, die geringer belastbar sind. Kinematisch besitzt das erfindungsgemäße Gelenk, das durch die Berührung der Regelflächen miteinander entsteht, vier Freiheitsgrade. Denn bei feststehender Regelfläche eines Gelenkteils kann die andere Regelfläche entlang der gegenseitigen Berührungslinie gleiten (erster Freiheitsgrad), es kann von einer Berührungslinie zur anderen Berührungslinie gleiten (zweiter Freiheitsgrad), es kann von einer Berührungslinie zur anderen Berührungslinie rollen (dritter Freiheitsgrad) und es kann um einen Punkt auf der momentan gemeinsamen Berührungslinie rotieren (vierter Freiheitsgrad). Das erfindungsgemäße künstliche Gelenk ist besonders für ein endoprothetisches Kniegelenk, aber auch in der Grundkonzeption für die Artikulationsflächen des menschlichen Ellenbogengelenks oder des menschlichen oberen Sprungsgelenks geeignet. Insbesondere wenn zwei der erfindungsgemäßen, paarweise angeordneten Gelenkteile parallel zu einem Gelenk verbunden ausgebildet sind, z.B. zur Schaffung eines menschlichen Kniegelenkes, wird ein ebener oder sphärischer Zwangslauf der über das Gelenk gekoppelten Gliedmaße zueinander erreicht.

Vorteilhafte Ausbildungen der Erfindung sind in den Unteransprüchen enthalten. Anhand der in den beiliegenden Figuren dargestellten Ausführungsbeispiele wird die Erfindung nunmehr näher erläutert. Es zeigen:

Fig. 1    eine Prinzipdarstellung der Zuordnung von auf Kontaktkreisen liegenden Kontaktlinien mit gemeinsamer Kontakttangente,

Fig. 2    eine Ansicht gemäß Fig. 1 in der zu den Ebenen der Kontaktkreise senkrechten Ebene,

Fig. 3    eine Ansicht gemäß Fig. 1 bei Abrollen der Kontaktlinien unter Berücksichtigung einer Schwenkbewegung um die jeweilige Tangente t mit entstehenden Gelenkflächen,

Fig. 4 und 5    Prinzipdarstellungen gemäß Fig. 1 und 2 bei einer alternativen Zuordnung der Kontaktkreise mit zugehörigen Kontaktlinien,

Fig. 6    eine Prinzipdarstellung der erfindungsgemäßen Artikulationsflächen,

Fig. 7    eine Ansicht eines erfindungsgemäßen künstlichen Gelenks als Endoprothese für das menschliche Kniegelenk und

Fig. 8    ein Kniegelenk für Vierbeiner.

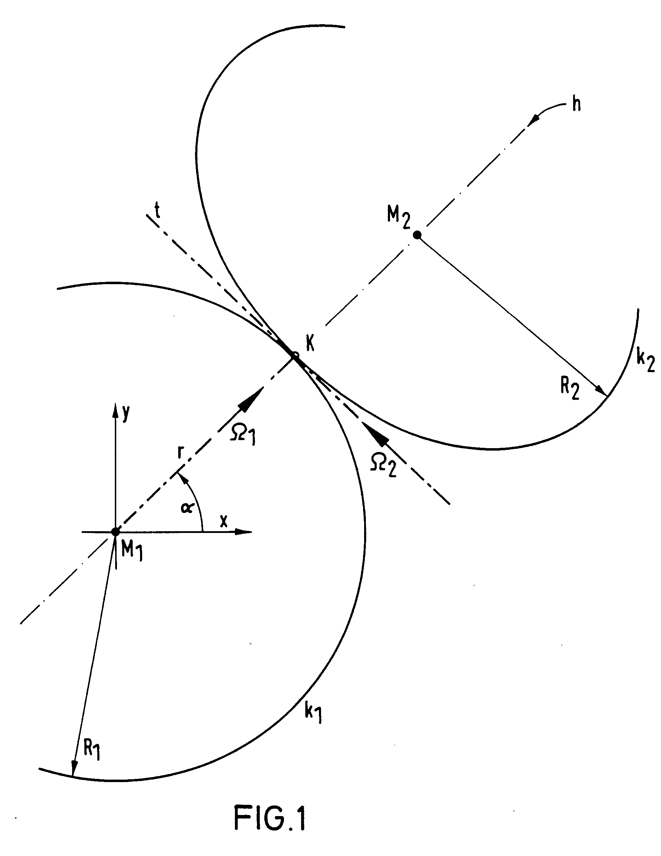

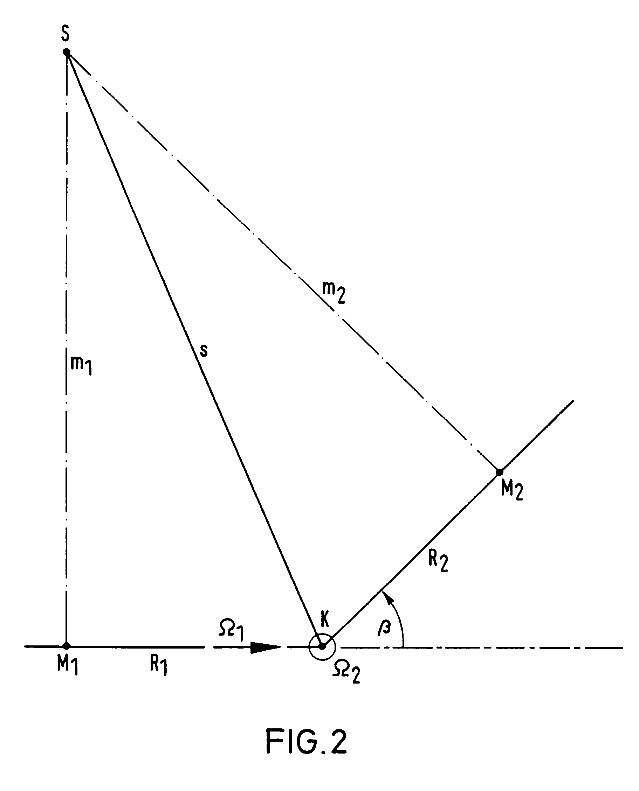

In der in Fig. 1 dargestellten Prinzipzeichnung sind jeweils eine Kontaktlinie $k_1$ und eine Kontaktlinie $k_2$ dargestellt. Diese Kontaktlinien $k_1$ und $k_2$ sind Teilabschnitte von Kontaktkreisen mit den Mittelpunkten $M_1$ und dem Radius $R_1$ bzw. dem Mittelpunkt $M_2$ und dem Radius $R_2$. Diese Kontaktlinien $k_1$ und $k_2$ sind auf in dieser Zeichnung nicht dargestellten Artikulationsflächen eines Gelenkkörpers 1 und eines Gelenkkörpers 2 ausgebildet, wobei der Gelenkkörper 1 beispielsweise ein Gelenkkopf/Gelenkpfanne sein kann und der Gelenkkörper 2 beispielsweise eine Gelenkpfanne/Gelenkkopf. Somit ist die Kontaktlinie $k_1$ einem Gelenkkörper 1 und die Kontaktlinie $k_2$ einem Gelenkkörper 2 zugeordnet. Wie Fig. 1 zu entnehmen ist, berühren sich die beiden Kontaktlinien $k_1$ und $k_2$ in einem Kontaktpunkt K, wenn die beiden Gelenkteile, d.h. die Gelenkkörper 1 und 2 zu einem Gelenk paarweise angeordnet sind. Durch den Kontaktpunkt K verläuft eine den beiden Kontaktlinien im Kontaktpunkt K gemeinsame Tangente t. Wie aus Fig. 2 ersichtlich ist, verlaufen durch die Mittelpunkte $M_1$ und $M_2$ jeweils, senkrecht zur Ebene der Kontaktkreise, Achsen $m_1$ und $m_2$, die sich in einem Schnittpunkt S schneiden. Dies ergibt sich aufgrund der Anordnung der Ebenen der Kontaktkreise derart, daß zwischen diesen Kontaktkreisen ein stumpfer Winkel ausgebildet ist. Im Falle, daß der zwischen den Ebenen der Kontaktkreise liegende Winkel 180° beträgt, schneiden sich die Achsen $m_1$ und $m_2$ im Unendlichen. Eine Verbindungslinie zwischen dem Kontaktpunkt K und dem Schnittpunkt S ist mit s gekennzeichnet. Mit dem Pfeil $\Omega_1$ ist der Bewegungsvektor einer momentanen Winkelgeschwindigkeit um eine momentane Achse r angegeben, die mit der Verbindungslinie zwischen dem Kreismittelpunkt $M_1$ und dem momentanen Kontaktpunkt $k_1$ übereinstimmt. Mit dem Pfeil $\Omega_2$ ist der Bewegungsvektor einer Schwenkbewegung angegeben um die Tangente t. Weiterhin ist in Fig. 2 ein Winkel β eingezeichnet, der der Komplementärwinkel zu dem zwischen den beiden Ebenen der Kontaktlinien eingeschlossenen Winkel ist. Der Bewegungsvektor $\Omega_1$ gibt somit an, daß die Kontaktlinien aufeinander abrollen können und der Bewegungsvektor $\Omega_2$ gibt an, daß dieser Abrollbewegung eine Schwenkbewegung der Kontaktlinien um die Tangente überlagert sein kann, wobei die Winkelgeschwindigkeit dieser Schwenkbewegung die Änderungsgeschwindigkeit des Winkels β ($\Omega_2$ = dß/dt) ist.

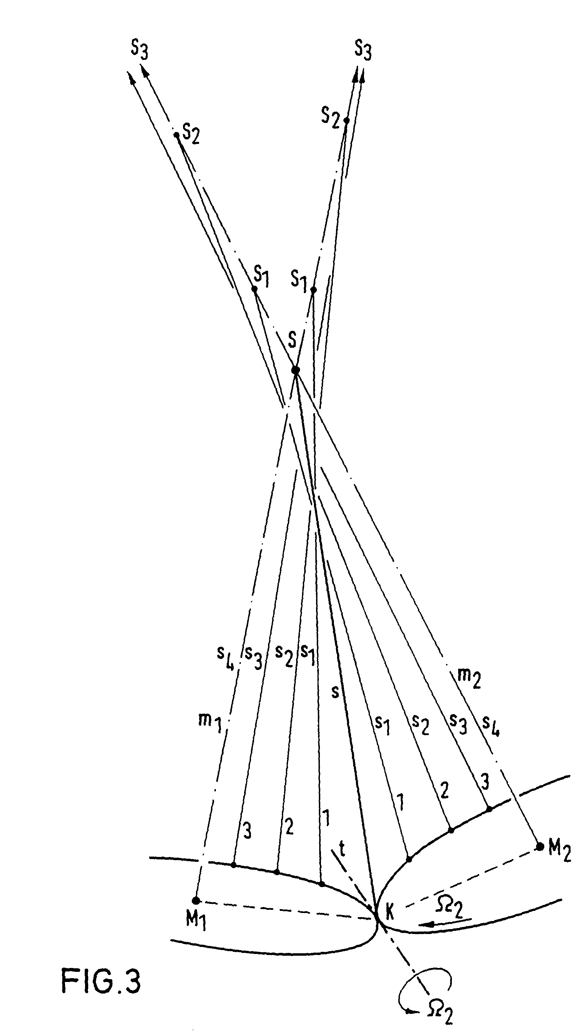

In Fig. 3 ist dargestellt, wie sich der Schnittpunkt S gemäß Fig. 2 und die Verbindungslinie s bei einer Abrollbewegung der Kontaktlinien $k_1$ und $k_2$ unter gleichzeitiger Durchführung einer Schwenkbewegung um die Tangente t verlagern. Gemäß Fig. 3 wird nun in jeder momentanen Stellung der Kontaktlinie $k_2$ der Schnittpunkt S der Achsen $m_1$ und $m_2$ bestimmt. Da die Kontaktlinie $k_2$ schwenkt, wandert der Schnittpunkt S mit der Bewegung sowohl entlang der Achse $m_1$ als auch der Achse $m_2$. In jeder Stellung wird der Schnittpunkt S mit dem momentanen Kontaktpunkt K verbunden. Die zugehörige Linie s ist die momentane Berührungslinie der beiden Artikulationsflächen, wobei durch die Gesamtheit der Berührungslinien s (Summe der Linien $s_1$, $s_2$, $s_3$...) zum bewegten und zum nicht bewegten Kontaktkreis bzw. zu den bewegten und nicht bewegten Kontaktlinien $k_1$ und $k_2$ die Regelflächen 3 und 4 gebildet werden, die einseitig sich an die Kontaktlinien $k_1$ und $k_2$ jeweils anschließen. Hierbei handelt es sich um gerade Berührungslinien s, die die Bereiche linienförmiger Kraftübertragung zwischen den gebildeten Regelflächen 3 und 4 während der gegenseitigen Artikulation darstellen.

Für die Herstellung der Regelflächen 3 und 4 mittels beispielsweise einer programmgesteuerten Fräsmaschine können folgende mathematische Bedingungen eingegeben werden:

Die beiden Winkelgeschwindigkeiten $\Omega_1$ und $\Omega_2$ sind Vektoren, deren Richtungen mit den Richtungen der momentanen Achsen r bzw. t übereinstimmen. In Vektorschreibweise heißt das:

$\underline{\Omega}_1 = \Omega_{10} \cdot (\underline{e}_x \cdot \cos\alpha + \underline{e}_y \cdot \sin\alpha)$ und $\underline{\Omega}_2 = \Omega_{20} \cdot (\underline{e}_x \cdot$

$\sin\alpha$ - $\underline{e}_y \cdot \cos\alpha$), da $\underline{\Omega}_1$ und $\underline{\Omega}_2$ aufeinander senkrecht stehen.

$\underline{e}_x$ = der Einheitsvektor in x-Richtung und
$\underline{e}_y$ = der Einheitsvektor in y-Richtung des ortsfesten Koordinatensystems, in dem der Kreis $k_1$ ruht.

Es sollen noch folgende Bedingungen eingeführt werden:
$\Omega_{10} = \Omega_0 \cdot \cos(\alpha + \delta)$ und $\Omega_{20} = \Omega_0 \cdot \sin(\alpha + \delta)$, was gleichbedeutend ist mit der Relation

$$\Omega_{10}/\Omega_{20} = ctg(\alpha + \delta).$$

Die Einführung dieser Bedingung ist möglich, da die momentane Rotation um die Achse t prinzipiell unabhängig von der momentanen Rotation um die Achse r erfolgen kann.

Die resultierende momentane Winkelgeschwindigkeitskomponente $\Omega$ (parallel zur y-x-Ebene) der Kreisscheibe $k_2$ setzt sich durch Vektoraddition der Winkelgeschwindigkeiten $\underline{\Omega}_1$ und $\underline{\Omega}_2$ zusammen.

Dadurch gilt für $\Omega$:

$\underline{\Omega} = \Omega_0 \cdot (\underline{e}_x \cdot \cos\delta + \underline{e}_y \cdot \sin\delta)$, was bedeutet, daß $\underline{\Omega}$ eine konstante Richtung in der y-x-Ebene aufweist. $\Omega_0$ kann frei gewählt werden. $\Omega_0$ gibt vor, wie schnell sich der Winkel $\alpha$ ändert.

Durch Wahl der Größen:

Anfangswert von $\beta$,

Phasenwinkel $\delta$ und

der Radien $R_1$ und $R_2$ der Kreise $k_1$ und $k_2$ wird die Gesamtheit der möglichen Regelflächenpaare eingestellt.

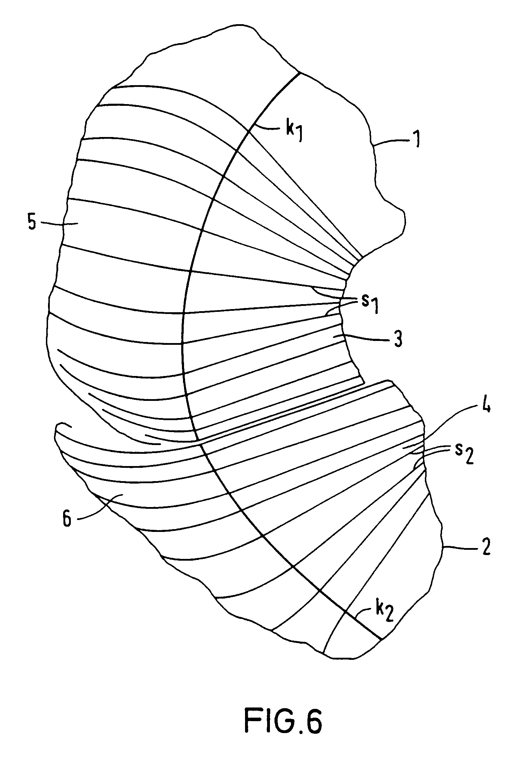

In Fig. 6 ist dargestellt, und zwar in perspektivischer Ansicht, wie die Artikulationsflächen eines Gelenkkörpers 1 und eines Gelenkkörpers 2 mittels der Kontaktlinien $k_1$ und $k_2$ und der aus der Vielzahl der Berührungslinien s gebildeten Regelflächen 3 und 4 ausgebildet sein können.

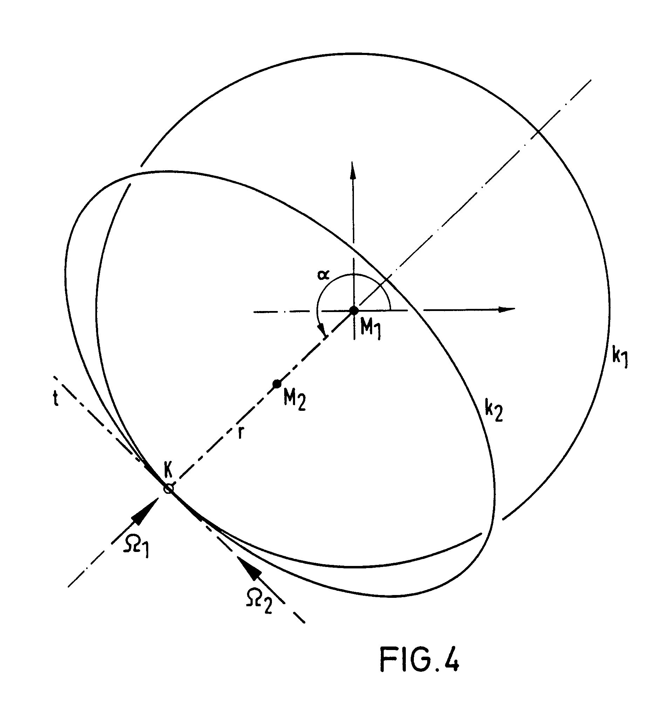

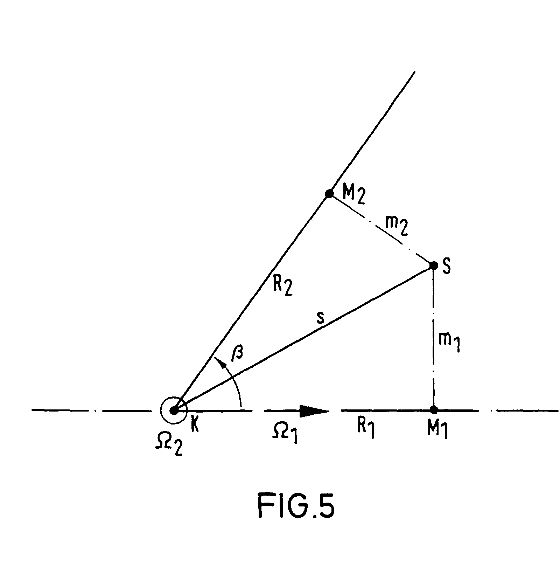

In den Fig. 4 und 5 sind gleiche Funktionselemente und -teile mit denselben Bezugsziffern wie in den Fig. 1 bis 3 dargestellt, wobei jedoch eine alternative Anordnung der Kontaktlinien $k_1$ und $k_2$ gewählt ist. Hierbei sind die die Kontaktlinien $k_1$ und $k_2$ aufweisenden Kontaktkreise derart zueinander angeordnet, daß sie zwischen sich einen spitzen Winkel einschließen. Dies bedeutet, daß sich die beiden Kontaktlinien $k_1$ und $k_2$ nicht von außen, d.h. in einer konvex-konvexen Lage berühren, sondern von innen, d.h. in einer Lage einer Konkavität mit einer Konvexität, wobei im dargestellten Ausführungsbeispiel der Kontaktkreis $K_1$ die Konkavität bildet und der Kontaktkreis $K_2$ die Konvexität.

Wie sich weiterhin aus Fig. 6 ergibt, besteht eine weitere zweckmäßige Ausgestaltung der Erfindung darin, daß auf der zu den Regelflächen 3 und 4 gegenüberliegenden Seite der Kontaktlinien $k_1$, $k_2$ die Berührungslinien s für die Kontaktlinien $k_1$, $k_1$ hinaus, vorteilhafterweise kreisbogenförmig, verlängert sind, so daß eine Wulst 5, 6 jeweils an den Gelenken 1, 2 ausgebildet wird. Dabei sind die Wulste 5, 6 derart geformt, daß sie sich bei der Artikulation der Gelenkkörper 1, 2 nicht berühren. Weiterhin sind die Wulste 5, 6 derart geformt, daß sie zwischen sich eine Querschnittsfläche jeweils einschließen über den gesamten Artikulationsbereich, die in ihrer Größe und Form in etwa konstant ist. Die Wulste 5, 6 bilden Bereiche der indirekten Kraftübertragung. In den Zwischenraum zwischen den Wulsten 5, 6 kann sich seitlich Gewebe einlagern, das beispielsweise eine ähnliche Funktion, wie die Menisken im natürlichen Kniegelenk ausüben kann. Da die Querschnittsfläche zwischen den Wulsten 5, 6 erfindungsgemäß etwa konstant ist, ergibt sich keine Quetschung des sich einlagernden Gewebes. Der Kreismittelpunkt der kreisbogenförmigen, die Wulstflächen bildenden Verlängerungen der Berührungslinien s ist kleiner/gleich dem Radius der Kontaktkreise der Kontaktlinien.

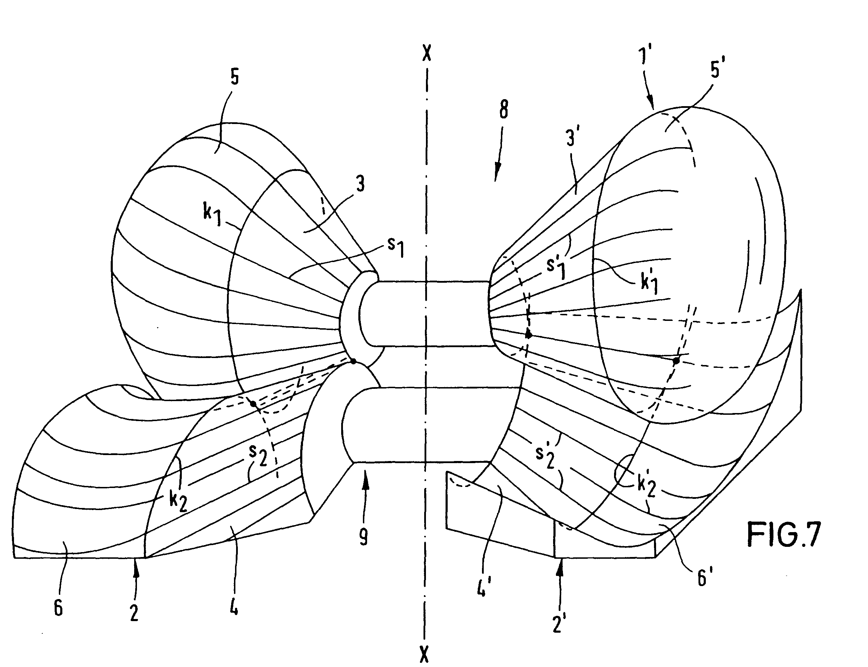

Wie in Fig. 7 dargestellt ist, kann das dort dargestellte erfindungsgemäße künstliche Gelenk als Endoprothese zum Ersatz des menschlichen Kniegelenkes (rechtes Knie in der Ansicht von vorne) aus der Parallelschaltung zweier erfindungsgemäßer Gelenkkörperpaare 1, 2; 1', 2' gebildet werden. Hierbei sind die Regelflächen 3, 4; 3', 4' jedes Gelenkkörperpaares derart zu einer Mittelebene X-X angeordnet, daß sie auf der der Mittelebene X-X zugekehrten Seite angeordnet sind. Die Gelenkkörper 1, 2; 1', 2' sind hierbei derart angeordnet, daß der Gelenkkörper 1 und der Gelenkkörper 1' starr miteinander verbunden sind und gemeinsam den femuralen Gelenkkopf 8 bilden. Die Gelenkkörper 2 und der Gelenkkörper 2' sind ebenfalls starr miteinander verbunden und bilden gemeinsam den tibialen Gelenkkörper 9 (tibiale Gelenkpfanne) des erfindungsgemäßen künstlichen Kniegelenks zum Ersatz des rechten Kniegelenks. Der femurale Gelenkkörper 1 und der tibiale Gelenkkörper 2 sind hierbei lateral angeordnet und der femurale Gelenkkörper 1' und der tibiale Gelenkkörper 2' sind medial angeordnet. Hierbei ist es insbesondere vorteilhaft, wenn die Regelflächen 3, 4 bzw. 3', 4' zur Gelenkaußenseite hin nach unten, d.h. in Richtung tibial geneigt verlaufen, so daß eventuell auftretende Abriebpartikel hierdurch aus dem Gelenkinneren nach außen abfließen können und durch diese dachförmige Zuordnung die Ab- bzw. Adduktions-Stabilität erhöht wird. Weiterhin ist es zweckmäßig, wenn die Flächen der Wulste 5, 6 bzw. 5', 6', d.h. die Flächen der indirekten Kraftübertragung, so gestellt sind, daß ein Herausfließen der Gelenk-Abriebpartikel weiterhin unterstützt wird.

Durch die erfindungsgemäße Parallelschaltung wird erreicht, daß sich die am femuralen Gelenkkopf 8 bzw. an der tibialen Gelenkpfanne 9 befestigten Gliedmaßen in einer zugeordneten Funktionsebene linear bewegen. Hierbei bildet die Kontaktlinienanordnung $K_1$, $K_2$ des lateralen Gelenks eine gestreckte dimere Kette, und die Kontaktlinienanordnung $K_1'$, $K_2'$ des medialen Gelenks bildet eine überschlagene dimere Kette, siehe Fig. 4.

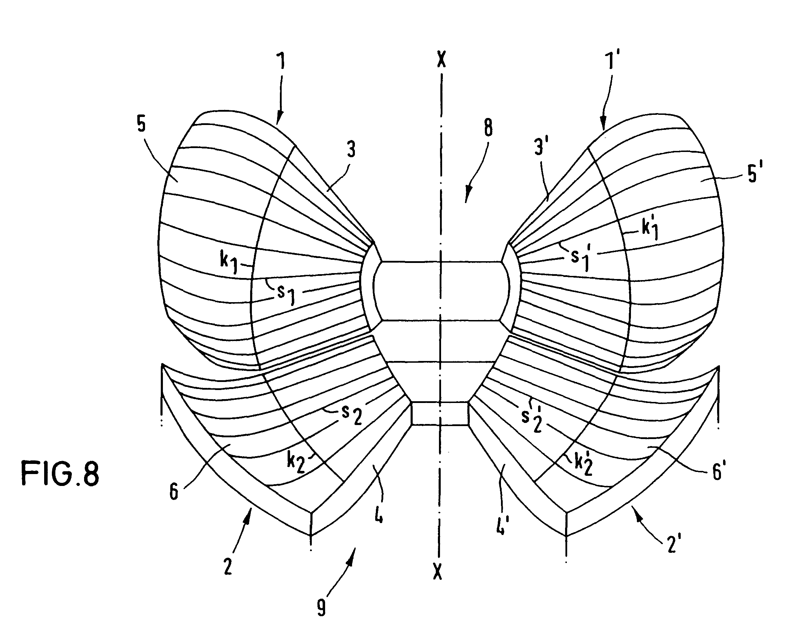

In Fig. 8 ist eine Ausbildung eines erfindungsgemäßen Gelenks als Kniegelenk eines Vierbeiners dargestellt. Hierbei ergibt sich eine Ausführung, wie in Fig. 7 erläutert. Jedoch besteht ein Unterschied zur Ausführung gemäß Fig. 7 darin, daß sowohl die Kontaktlinienanordnung $K_1$, $K_2$ als auch die Kontaktlinienanordnung $K_1'$, $K_2'$ jeweils eine gestreckte dimere Kette, siehe Fig. 3, bilden.

**Patentansprüche**

1. Künstliches Gelenk, insbesondere Endoprothese zum Ersatz natürlicher Gelenke, bestehend aus mindestens zwei künstlichen Gelenkteilen mit gekrümmten Artikulationsflächen, wobei auf jeder der Artikulationsflächen eine kreisbogenförmige Kontaktlinie (k) ausgebildet ist, die jeweils ein Teilabschnitt eines in einer Ebene liegenden Kontaktkreises mit einem Mittelpunkt (M) ist, wobei die Artikulationsflächen derart als Paar zueinander angeordnet sind, daß die Kontaktlinien (k) aufeinander abrollen können, sowie senkrecht zu der Ebene der Kontaktkreise durch deren Mittelpunkt (M) verlaufende Achsen (m) sich in einem Schnittpunkt (S) schneiden, sowie einseitig an die Kontaktlinien (k) aus einer Vielzahl von geraden Berührungslinien gebildete die Artikulationsflächen darstellende Regelflächen (3, 4) ausgeformt sind, wobei die Berührungslinien auf momentanen Verbindungslinien (s) der während der Abrollbewegung auftretenden momentanen Kontaktpunkten (K) mit den momentanen Schnittpunkten (S) liegen, welche sich durch eine Schwenkbewegung der Kontaktlinien (k) mit einer Winkelgeschwindigkeit (Ω) um eine gemeinsame Tangente (t) der Kontaktlinien (k) durch die momentanen Kontaktpunkte (K) ergeben.

2. Künstliches Gelenk nach Anspruch 1, **dadurch gekennzeichnet**, daß die Ebenen der Kontaktkreise zwischen sich einen stumpfen oder einen spitzen Winkel einschließen.

3. Künstliches Gelenk nach Anspruch 1 oder 2, **dadurch gekennzeichnet**, daß auf der den Regelflächen (3, 4) gegenüberliegenden Seite der Kontaktlinien (k) die Berührungslinien (s) derart insbesondere in Kreisbögen verlängert sind, daß Wulste (5, 6) ausgebildet werden, wobei die einander gegenüberliegenden Wulste (5, 6) der zugehörigen Artikulationsflächen keinen Berührungskontakt besitzen und die zwischen ihnen vorhandenen Querschnittsflächen im gesamten Artikulationsbereich in Größe und Form im wesentlichen konstant sind.

4. Künstliches Gelenk nach einem der Ansprüche 1 bis 3, **dadurch gekennzeichnet**, daß die kraftübertragenden Regelflächen (3, 4) derart schräg angeordnet sind, daß ein stetiges Abfließen von Abriebpartikeln erfolgt.

5. Künstliches Gelenk nach einem der Ansprüche 1 bis 4, **dadurch gekennzeichnet**, daß zwei Gelenkteilpaare parallel zueinander angeordnet sind und gemeinsam eine Endoprothese für ein menschliches Knie bilden.

**Claims**

1. An artificial joint, especially an endoprosthesis to replace a natural joint, comprising at least two artificial joint components with curved articulation surfaces, whereby a circular arc shaped contact line (k) is formed on each of the articulation surfaces, which in each case is a segment of a contact circle lying in a plane with a centre point (M), whereby the articulation surfaces are arranged with each other as a pair, such that the contact lines (k) can roll on each other, and both, perpendicular to the plane of the contact circle the axes (m) running through their centre point (M) meet at a point of intersection (S), and on one side of the contact lines (k) controlling surfaces (3, 4) are formed from a multiplicity of straight lines of contact representing the articulation surfaces, whereby the lines of contact lie on instantaneous joint lines (s) of the instantaneous contact points (K) arising during the rolling movement with the instantaneous intersection points (S), which result from a swivelling movement of the contact lines (k) with an angular speed (Ω) about a common tangent (t) of the contact lines (k) through the instantaneous contact points (K).

2. An artificial joint according to Claim 1, **characterised in that** the planes of the contact circles include between them an obtuse or an acute angle.

3. An artificial joint according to Claim 1 or Claim 2, **characterised in that** on the opposite side of the contact lines (k) to the controlling surfaces (3, 4) the lines of contact (s) are extended especially in the arc of a circle such that protuberances (5, 6) are formed, whereby the opposing protruberances (5, 6) of the associated articulation surfaces have no touching contact and the cross-sectional surfaces present between them are constant in size and form over the whole range of articulation.

**4.** An artificial joint according to one of the Claims 1 to 3, **characterised in that** the force transmitting control surfaces (3, 4) are arranged so inclined that a steady outflow of frictionally removed particles occurs.

**5.** An artificial joint according to one of the Claims 1 to 4, **characterised in that** two joint component pairs are arranged parallel to each other and together form an endoprosthesis for a human knee.

**Revendications**

**1.** Articulation artificielle, en particulier endoprothèse pour le remplacement d'articulations naturelles, constituée d'au moins deux parties articulaires artificielles avec des surfaces articulaires courbes, une ligne de contact en arc de cercle (k) qui est à chaque fois un segment d'un cercle de contact situé dans un plan et pourvu d'un centre (M) étant formée sur chacune des surfaces articulaires, les surfaces articulaires étant disposées par paires associées, de façon que les lignes de contact (k) puissent rouler l'une sur l'autre, des axes (m) passant par le centre (M) des cercles de contact, perpendiculairement à leur plan, se coupant en un point d'intersection (S), et des surfaces réglées (3, 4) représentant les surfaces articulaires et formées par une pluralité de génératrices droites étant conformées d'un côté des lignes de contact (k), les génératrices se trouvant sur des lignes de jonction momentanée (s) des points de contact momentané (K), engendrés durant le mouvement de roulement, avec les points d'intersection momentanée (S), lesquels résultent d'un mouvement pivotant des lignes de contact (k) à une vitesse angulaire (Ω) autour d'une tangente commune (t) aux lignes de contact (k) passant par les points de contact momentané (K).

**2.** Articulation artificielle selon la revendication 1, **caractérisée en ce que** les plans des cercles de contact forment entre eux un angle obtus ou un angle aigu.

**3.** Articulation artificielle selon la revendication 1 ou 2, **caractérisée en ce que**, sur le côté des lignes de contact (k) situé à l'opposé des surfaces réglées (3, 4), les génératrices (s) sont prolongées en particulier par des arcs de cercle, de façon à former des bourrelets (5, 6), les bourrelets en vis-à-vis (5, 6) des surfaces articulaires associées n'étant pas en contact, et les aires de section existant entre eux étant sensiblement constantes en taille et en forme dans toute la zone articulaire.

**4.** Articulation artificielle selon l'une des revendications 1 à 3, **caractérisée en ce que** les surfaces réglées (3, 4) transmettant la force sont disposées de manière inclinée, de façon à obtenir un écoulement continu de particules d'abrasion.

**5.** Articulation artificielle selon l'une des revendications 1 à 4, **caractérisée en ce que** deux paires de parties articulaires sont disposées parallèlement l'une à l'autre et forment conjointement une endoprothèse pour un genou humain.

FIG.1

FIG.2

FIG.3

EP 0 831 758 B1

9

FIG.4

FIG.5

FIG.6

FIG.7

FIG.8